# EUROPÄISCHE PATENTSCHRIFT

(11) **EP 1 344 493 B1**
(45) Veröffentlichungstag und Bekanntmachung des Hinweises auf die Patenterteilung: **02.09.2009**
(21) Anmeldenummer: 02005629.7
(22) Anmeldetag: 12.03.2002
(51) Int. Cl.: A61B 17/17, A61F 2/46

(54) **Intrumentarium zum Einsetzen einer Zwischenwirbelprothese**
Instument set for insertion of intervertebral prostheses
Ensemble d'instrument d'insertion d'une prothese intervertébrale

(43) Veröffentlichungstag der Anmeldung: 17.09.2003
(73) Patentinhaber: Cervitech, Inc., Rockaway, NJ 07866 (US)
(72) Erfinder: Keller, Arnold, 23863Kayhude (DE); McAfee, Paul C. M.D., Baltimore, MD 21204 (US)
(74) Vertreter: Glawe, Delfs, Moll

(56) Entgegenhaltungen:
- WO-A-01/62166
- US-A- 6 159 214
- US-A1- 2001 010 002

## Beschreibung

Beim Einsetzen einer Zwischenwirbelprothese zum Ersatz einer Bandscheibe arbeitet der Operateur in einem sehr unübersichtlichen Operationsfeld in unmittelbarer Nähe bedeutender Nerven- und Blutbahnen. Die Gefahr, diese zu beschädigen, ist groß. Die Erfindung sucht daher nach einem Mittel, die Führung einzelner Operationsschritte unabhängig von unmittelbarer Sicht zu machen. Das gilt insbesondere für Operationen an der Halswirbelsäule, weil dort die Wirbelabmessungen sehr klein und die Abstände zu sensiblen Nachbarbereichen besonders gering sind.

*In bekannten Instrumentarien für das Einsetzen eines Cages, der zur Fusion zweier benachbarter Wirbelkörper dienen soll (*US-A-6,159,214*,* U5-A-2001/0010002*,* WO 01/62166*), sind Instrumente enthalten, die zur Führung weiterer Instrumente dienen und gegebenenfalls die beteiligten Wirbelkörper im Verhältnis zueinander festlegen. Für ihre Positionierung sind Distraktorklingen vorgesehen, die vor dem Entfernen der Bandscheibe in diese eingestoßen werden und dabei die beteiligten Wirbelkörper auf einen vorbestimmten Abstand bringen. Da ihre Klingenebene in einer Sagittalebene liegt, ist ihre Stellung ein für allemal auf diejenige Ebene festgelegt, in der sie eingestoßen wurden. Eine genaue und gegebenenfalls auch nachträglich korrigierbare Lagebestimmung ist damit nicht möglich. Das ist für die Implantation von Cages auch nicht erforderlich, weil sie nicht sehr genau positioniert werden müssen.*

*Im Gegensatz dazu müssen Zwischenwirbelprothesen, die zum Ersatz der gelenkigen Funktion einer Bandscheibe vorgesehen sind, sehr genau positioniert werden, weil eine Fehlpositionierung zu erheblichen Beschwerden führen kann.*

*Der Erfindung liegt daher die Aufgabe zugrunde, ein Instrumentarium zu schaffen, das eine genaue Positionierung im Verhältnis zu den Wirbelkörpern ermöglicht.*

*Die erfindungsgemäße Lösung besteht* darine, *daß für die korrekte Positionierung einer Führungseinrichtung ein Justierinstrument vorgesehen ist,* das zunächst an charakteristischen Oberflächenteilen der Wirbelkörper plaziert wird, um die Justierung der Führungseinrichtung zu ermöglichen. *Da* es bei dem Einsetzen einer Zwischenwirbelprothese stets darum geht, die genaue Lage der die Bandscheibe einschließenden Wirbelflächen zu ermitteln, *verwendet* die Erfindung ein Justierinstrument, das eine Zwischenwirbelplatte umfaßt. Nachdem die Bandscheibe und gegebenenfalls die ventralen Randzacken des oberen Wirbelkörpers entfernt wurden, wird diese Zwischenwirbelplatte eingesetzt und gibt dann einen genauen Hinweis auf die Lage der Wirbelkörperoberflächen, zwischen denen die Prothese plaziert werden soll. Das Justierinstrument weist Justierflächen auf, die es gestatten, entsprechend der Lage der Zwischenwirbelplatte die Führungseinrichtung an einen der betroffenen Wirbel oder an beide Wirbel anzusetzen. Man kann dann sicher sein kann, daß sie exakt positioniert ist. Danach wird das Justierinstrument entfernt, und die Führungseinrichtung sichert die Positioniergenauigkeit des anschließenden Instrumenteneinsatzes.

Die Zwischenwirbelplatte liegt zwischen den Wirbeln und kann daher nicht unmittelbar zur Justierung der Führungseinrichtung beitragen. Es sind deshalb an der Zwischenwirbelplatte Justierflächen angeordnet, die mit der Führungseinrichtung zusammenwirken. Besonders vorteilhaft ist die Ausführung dieser Justierflächen als Justierstange, die nach ventral von der Zwischenwirbelplatte vorragt. Dies ermöglicht es dem Operateur, die zusammenwirkenden Justierflächen der Stange und der Führungseinrichtung in einem vorderen, übersichtlichen Teil des Operationsfeldes zusammenzustecken, statt in der unübersichtlichen Tiefe. Die Führungseinrichtung wird dann entlang der Justierstange in die Tiefe des Operationsfeldes geführt, bis sie den bzw. die Wirbel erreicht, an denen sie zu befestigen ist. Diese Befestigung findet statt, solange die Führungseinrichtung noch von dem Justierinstrument positioniert ist. Danach wird das Positionierinstrument entfernt.

Das Entfernen des Positionierinstruments ist unproblematisch, wenn die Führungseinrichtung lediglich an einem Wirbelkörper befestigt ist und das Justierinstrument bzw. dessen Justierstange nur von einer Seite her berührt. Bevorzugt wird aber eine Ausführung, bei welcher die Führungseinrichtung das Justierinstrument bzw. dessen Justierstange umfaßt. Dies ist insbesondere dann der Fall, wenn die Führungseinrichtung an beiden Wirbelkörpern zu befestigen ist. Das Justierinstrument muß dann durch eine Öffnung der Führungseinrichtung herausgenommen werden. Diese Öffnung muß also mindestens so groß sein wie das Justierinstrument. Eine solche Öffnungsgröße kann für die Justier- und Führungsaufgaben unzweckmäßig sein. Deshalb schlägt die Erfindung vor, zwischen den Justierflächen (der Justierstange) des Justierinstruments und den Justierflächen des Führungsinstruments ein Justierzwischenstück vorzusehen. Dieses weist einerseits Flächen auf, die justiergenau auf den Justierflächen des Justierinstruments (der Justierstange) gleiten und andererseits Flächen, die mit den Justierflächen der Befestigungseinrichtung zusammenwirken. Ferner kann eine Lehre vorgesehen sein, die innerhalb der Öffnung der Führungseinrichtung an diese angesetzt werden kann und die die Führungsflächen für die Instrumente bildet.

Die Erfindung wird im folgenden näher unter Bezugnahme auf die Zeichnung erläutert, die ein vorteilhaftes Ausführungsbeispiel veranschaulicht. Darin zeigen:
- Fig. 1: Die schematische perspektivische Ansicht von schräg ventral eines Paares von Halswirbeln mit einem Justierinstrument,
- Fig. 2: dieselbe Ansicht mit eingesetztem Justierinstrument,
- Fig. 3: die Führungseinrichtung mit Justierzwischenstück,
- Fig. 4: die Ansicht gemäß Fig. 1 mit eingesetztem Justierinstrument und angesetzter Führungseinrichtung und
- Fig. 5: die Ansicht gemäß Fig. 1 ohne Justierinstrument, mit angesetzter Führungseinrichtung und Fräslehre.

Im Zwischenwirbelraum 1 der Wirbelkörper 2 soll eine Zwischenwirbelprothese eingesetzt werden. Dafür müssen die einander zugewendeten Flächen der Wirbelkörper 2 bearbeitet werden. Sensible Bereiche sind eng benachbart (die Figuren zeigen die realen Verhältnisse bei der Halswirbelsäule etwa um den Faktor 2 vergrößert). Um die mit der Bearbeitung verbundenen Gefahren zu vermindern, soll eine Bearbeitungslehre 3 verwendet werden, die in vorbestimmter Position an den Wirbelkörpern 2 zu befestigen ist. Dafür dient die Führungseinrichtung 4. Sie ist rahmenförmig mit einer Öffnung 6, die passend zu einem Ansatz 7 an der Lehre 3 ausgebildet ist. Mittels Stiften 8 ist sie an den Wirbelkörpern 2 zu befestigen. Dies muß mit hoher Genauigkeit geschehen.

Dafür ist das Justierinstrument 10 vorgesehen. Es umfaßt eine Zwischenwirbelplatte 11 und eine damit fest verbundene Justierstange 12. Die Zwischenwirbelplatte 11 hat eine Flächenausdehnung, die wenig geringer ist als die Flächenausdehnung des Zwischenwirbelraums 1. Ihre Dicke ist in der Regel nicht größer als die einer Zwischenwirbelprothese. Sie ist jedenfalls so groß, daß sie nach Entfernung der Bandscheibe in den Zwischenwirbelraum 1 eingesetzt werden kann und dort durch die zwischen den Wirbelkörpern 2 herrschende, natürliche Spannung in der ihr erteilten Lage festgehalten wird. Sie besitzt eine Querbohrung 13 und eine AP-Bohrung 14, die auch die Führungsstange 12 durchzieht. Diese Bohrungen gestatten es, die Zwischenwirbelplatte 11 unter Röntgenkontrolle genau im Zwischenwirbelraum 1 zu positionieren. Die Justierstange 12 hat dann eine genau definierte Lage im Verhältnis zu den die Zwischenwirbelplatte 11 einschließenden Wirbeloberflächen.

Um die Befestigungseinrichtung 4 auf der Justierstange 12 justieren zu können, ist das Justierzwischenstück 18 vorgesehen, daß - ebenso wie die Fräslehre 3 - einen in die Öffnung 6 passenden Ansatz 7 trägt. Es enthält eine zum Außendurchmesser der Justierstange 12 passende Bohrung 19. Die Teile werden zunächst zusammengesteckt, so daß sie aufgrund ihrer Reibung oder anderer geeigneter Haftmittel hinreichend fest für die Manipulation miteinander verbunden sind. Sie werden dann mit der Bohrung 19 auf die Stange 14 gesetzt und können an ihr entlang gleiten, bis die Führungseinrichtung 4 an den ventralen Stirnflächen der Wirbelkörper 2 anliegt.

Dadurch, daß die Führungseinrichtung 4 über das Justierzwischenstück 18 auf der Justierstange 12 sitzt, ist Gewähr dafür vorhanden, daß sie genau die richtige Höhe zu den Wirbelendflächen hat, die den Zwischenwirbelraum 1 begrenzen. Zwar kann sie sich um die Stange 12 drehen; jedoch ist in dieser Beziehung keine wesentliche Fehleinstellung möglich. Will man auch diese sicher vermeiden, so verwendet man eine Justierstange 12 und eine dazu passende Öffnung 19, die nicht zylindrisch, sondern prismatisch geformt sind, beispielsweise einen Rechteckquerschnitt haben.

Das Justierzwischenstück 18 wäre entbehrlich, wenn die Justierstange 12 eine Fortsetzung der Zwischenwirbelplatte 11 mit gleichem Querschnitt wäre und die Öffnung 6 in der Befestigungseinrichtung gleichfalls diesen Querschnitt hätte. Dann wäre jedoch das große Ausmaß der Justierstange 12 möglicherweise hinderlich. Auch ist es besser, wenn die Größe der Öffnung 6 in der Justiereinrichtung 4 unabhängig von der Größe der Zwischenwirbelplatte 11 bemessen werden kann.

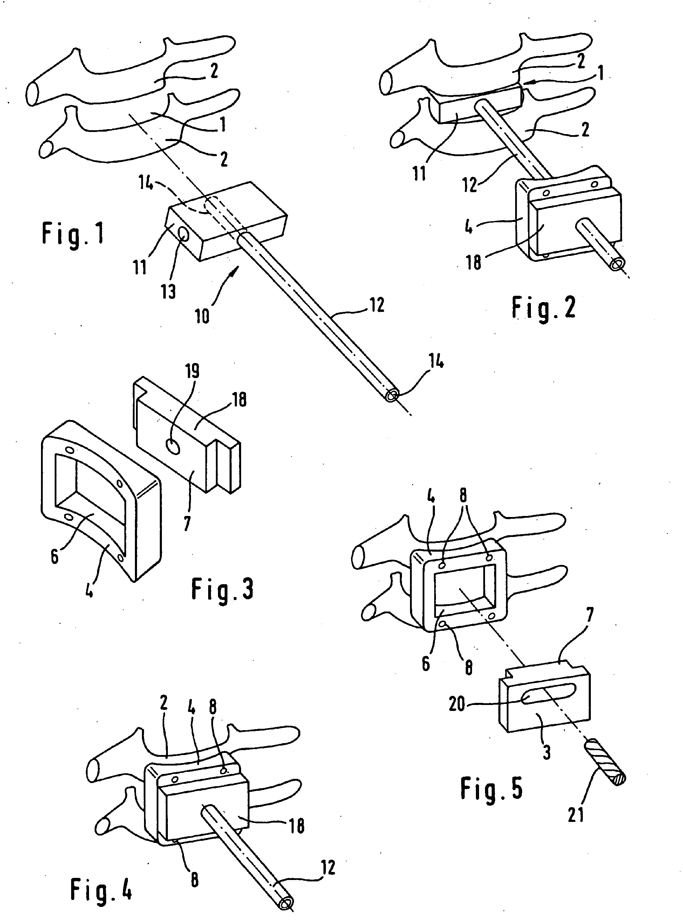

Sobald die Führungseinrichtung 4 den vorgesehenen Platz an den Wirbeln 2 erreicht hat, wie es in Fig. 4 dargestellt ist, wird sie durch ihre Bohrungen 8 mittels feiner Knochenschrauben an den Wirbelkörpern 2 befestigt. Das Justierzwischenstück 18 und die Zwischenwirbelplatte 11 können nun durch die Öffnung 6 hindurch entfernt werden. Damit stellt sich der in Fig.5 dargestellte Zustand ein.

In die Öffnung 6 der Führungseinrichtung 4 können nun beliebige Bearbeitungslehren 3 eingesetzt werden, die in Abstimmung auf die jeweils verwendeten Bearbeitungswerkzeuge Ausschnitte 20 zu deren Führung enthalten. Beispielsweise kann der in Fig. 5 dargestellte Schlitz 20 zur Führung eines Walzenfräsers dienen, der zur Bearbeitung der Prothesenansatzfläche des oberen Wirbelkörpers 2 verwendet wird.

## Patentansprüche

1. Instrumentarium zum Einsetzen einer zervikalen Zwischenwirbelprothese zum Ersatz einer Bandscheibe, das eine an wenigstens einem Wirbelkörper (2) zu befestigende Führungseinrichtung (4) für ein Instrument oder ein Prothesenteil sowie ein Justierinstrument (10) zum Justieren der Führungseinrichtung (4) bei deren Anbringen am Wirbelkörper (2) umfaßt, das eine in den Zwischenwirbelraum einzuschiebende Zwischenwirbelplatte (11) aufweist, **dadurch gekennzeichnet, daß** das Justierinstrument mindestens eine in AP-Richtung ausgerichtete Röntgenkontrollmarke (14) umfaßt.

2. Instrumentarium nach Anspruch 1, **dadurch gekennzeichnet, daß** das Justierinstrument (10) eine sich nach ventral erstreckende Justierstange (12) für die Führungseinrichtung (4) aufweist.

3. Instrumentarium nach Anspruch 1 oder 2, **dadurch gekennzeichnet, daß** die Führungseinrichtung (4) zur Befestigung an beiden durch die Zwischenwirbelprothese zu verbindenden Wirbelkörpern (2) ausgebildet ist und eine Öffnung (6) aufweist, die größer als die Zwischenwirbelplatte (11) ist.

4. Instrumentarium nach Anspruch 3, **dadurch gekennzeichnet, daß** ein an die Führungseinrichtung (4) ansetzbares, auf der Justierstange (12) verschiebbares Justierzwischenstück (18) vorgesehen ist.

5. Instrumentarium nach einem der Ansprüche 1 bis 4, **dadurch gekennzeichnet, daß** eine an die Führungseinrichtung (4) ansetzbare Lehre (3) für ein Bearbeitungsinstrument vorgesehen ist.

## Claims

1. Instrument set for fitting a cervical intervertebral prosthesis as a replacement for an intervertebral disk, comprising a guide device (4) for an instrument or a prosthesis part, which guide device (4) is to be secured on at least one vertebral body (2), and an adjustment instrument (10) for adjusting the guide device (4) when the latter is arranged on the vertebral body (2), which adjustment instrument (10) has an intervertebral plate (11) which is to be inserted into the intervertebral space, **characterized in that** the adjustment instrument comprises at least one X-ray control mark (14) which is aligned in the AP direction.

2. Instrument set according to Claim 1, **characterized in that** the adjustment instrument (10) has an adjustment rod (12) for the guide device (4), said adjustment rod (12) extending in the ventral direction.

3. Instrument set according to Claim 1 or 2,
**characterized in that** the guide device (4) is designed to be secured to both the vertebral bodies (2) which are to be connected by the intervertebral prosthesis, and it has an opening (6) which is greater than the intervertebral plate (11).

4. Instrument set according to Claim 3, **characterized in that** an intermediate adjustment piece (18) is provided which can be attached to the guide device (4) and which is displaceable on the adjustment rod (12).

5. Instrument set according to one of Claims 1 to 4, **characterized in that** a gauge (3) which can be attached to the guide device (4) is provided for a working instrument.

## Revendications

1. Ensemble d'instrument pour insérer une prothèse intervertébrale cervicale en vue de remplacer un disque, qui comprend un dispositif de guidage (4) à fixer sur au moins un corps de vertèbre (2) pour un instrument ou une partie de prothèse ainsi qu'un instrument d'ajustement (10) pour ajuster le dispositif de guidage (4) lors de son placement sur le corps de vertèbre (2), qui présente une plaque intervertébrale (11) à insérer dans l'intervalle intervertébral, **caractérisé en ce que** l'instrument d'ajustement comprend au moins une marque de contrôle aux rayons X (14) orientée dans la direction AP.

2. Ensemble d'instrument selon la revendication 1, **caractérisé en ce que** l'instrument d'ajustement (10) présente une tige d'ajustement (12) s'étendant en direction du côté ventral, pour le dispositif de guidage (4).

3. Ensemble d'instrument selon la revendication 1 ou 2, **caractérisé en ce que** le dispositif de guidage (4) est réalisé en vue d'être fixé aux deux corps de vertèbre (2) à relier par la prothèse intervertébrale et présente une ouverture (6), qui est plus grande que la plaque intervertébrale (11).

4. Ensemble d'instrument selon la revendication 3, **caractérisé en ce qu'**il est prévu une pièce intermédiaire d'ajustement (18) pouvant coulisser sur la tige d'ajustement (12) et applicable sur le dispositif de guidage (4).

5. Ensemble d'instrument selon l'une quelconque des revendication 1 à 4, **caractérisé en ce qu'**il est prévu un gabarit (3) pour un instrument de traitement, applicable sur le dispositif de guidage (4).
